# EUROPÄISCHE PATENTSCHRIFT

(11) **EP 1 414 336 B1**
(45) Veröffentlichungstag und Bekanntmachung des Hinweises auf die Patenterteilung: **26.05.2010**
(21) Anmeldenummer: 02754464.2
(22) Anmeldetag: 01.08.2002
(51) Int. Cl.: A61B 5/00, G01N 21/64

(54) **SYSTEM FÜR DIE FLUORESZENZDIAGNOSTIK**
FLUORESCENCE DIAGNOSTIC SYSTEM
SYSTEME DE DIAGNOSTIC PAR FLUORESCENCE

(30) Priorität: 09.08.2001 DE 10139239; 23.11.2001 DE 10157575
(43) Veröffentlichungstag der Anmeldung: 06.05.2004
(73) Patentinhaber: Biocam GmbH, 93053 Regensburg (DE)
(72) Erfinder: PLÄN, Thomas, 93093 Donaustauf (DE)
(74) Vertreter: Graf, Helmut
(86) Internationale Anmeldenummer: PCT/DE2002/002818
(87) Internationale Veröffentlichungsnummer: WO 2003/016878

(56) Entgegenhaltungen:
- EP-A- 1 116 473
- US-A- 4 773 097
- US-A- 5 749 830
- PATENT ABSTRACTS OF JAPAN vol. 1997, no. 05, 30. Mai 1997 (1997-05-30) & JP 09 024052 A (FUJI PHOTO FILM CO LTD), 28. Januar 1997 (1997-01-28)

## Beschreibung

Die Erfindung bezieht sich auf ein System für die Fluoreszenzdiagnostik gemäß Oberbegriff Patentanspruch 1.

Die Fluoreszenzdiagnostik ist als ein noch experimentielles, klinisch nicht zugelassenes Verfahren grundsätzlich bekannt, welches u.a. auch für die quantitative Früherkennung einer Vielzahl von präkanzerosen/dysplastischen Veränderungen an Geweben sehr vielversprechend ist. Die Fluoreszenzdiagnostik macht sich die Stoffwechselunterschiede beispielsweise im Porphyrinstoffwechsel zwischen Zellen in dysplastischem/tumorösem Gewebe und Zellen im normalen Gewebe zunutze.

Aus der Druckschrift US 5,749,830 ist eine Vorrichtung zur Fluoreszenzdiagnostik und Endoskopie bekannt, bei der ein Gewebeabschnitt mit einem von einer durch eine Lasereinheit gebildeten Anregungslichtquelle erzeugten Anregungslicht beaufschlagt wird, und zwar zur Erzeugung einer Fluoreszenz. Über ein mechanisches Filterrad wird das Anregungslicht periodisch unterbrochen und im Blockierungszeitraum wird der Gewebeabschnitt abwechselnd mit rotem, grünem und blauem Licht beaufschlagt, und zwar einzeln nacheinander. Über eine externe Kameraeinheit mit einem opto-elektrischen Bildwandler werden sowohl ein Fluoreszenzbild als auch jeweils ein rotes, grünes und blaues Bild abwechselnd aufgenommen. Die Reihenfolge und Aufnahmedauer ist durch die konstruktive Ausbildung der vorgeschalteten Filtereinheiten streng vorgegeben. Aus den drei Bildern wird ein RGB-Farbbild zusammengesetzt, welches über eine Monitoreinheit zusammen mit dem Fluoreszenzbild darstellbar ist.

Aufgabe der Erfindung ist es, ein System aufzuzeigen, mit dem eine verbesserte und eindeutigere Fluoreszenzdiagnostik möglich ist. Zur Lösung dieser Aufgabe ist ein System entsprechend dem Patentanspruch 1 ausgebildet.

Das erfindungsgemäße System dient zur Visualisierung von Fluoreszenzfarbstoffen zur Fluoreszenzdiagnostik u.a. von humanen Tumoren, ist speziell auch für eine hochsensitive, quantitative Früherkennung einer Vielzahl von präkanzerosen/dysplastischen Veränderungen geeignet und nutzt dabei beispielsweise den Unterschied im Porphyrinstoffwechsel bei Zellen in dysplastischem/tumorösem Gewebe im Vergleich zu Zellen im normalen Gewebe. Das erfindungsgemäße System gestattet weiterhin ein sehr vereinfachtes Arbeiten bei hoher Sensitivität und eindeutigen Ergebnissen.

Als Fluoreszenzfarbstoffe eignen sich u.a. Fluorophore, die Licht im sichtbaren Spektralbereich oder nahen Infrarotbereich absorbieren und Licht im nahen Infrarotbereich emittieren. Zu diesen Fluorophoren zählen insbesondere auch exogene Farbstoffe, d. h. von außen in das Gewebe eingebrachte Farbstoffe, wie z. B. Indocyaningrün und verschiedene Porphycene, aber auch endogene Farbstoffe, d.h. im Gewebe erzeugte Farbstoffe, wie z. B. 5-Aminolävulinsäure induzierte Porphyrine (insbesondere Protoporphyrin IX) usw.

Die Erfindung wird im folgenden anhand der Figuren an einem Ausführungsbeispiel näher erläutert. Es zeigen:

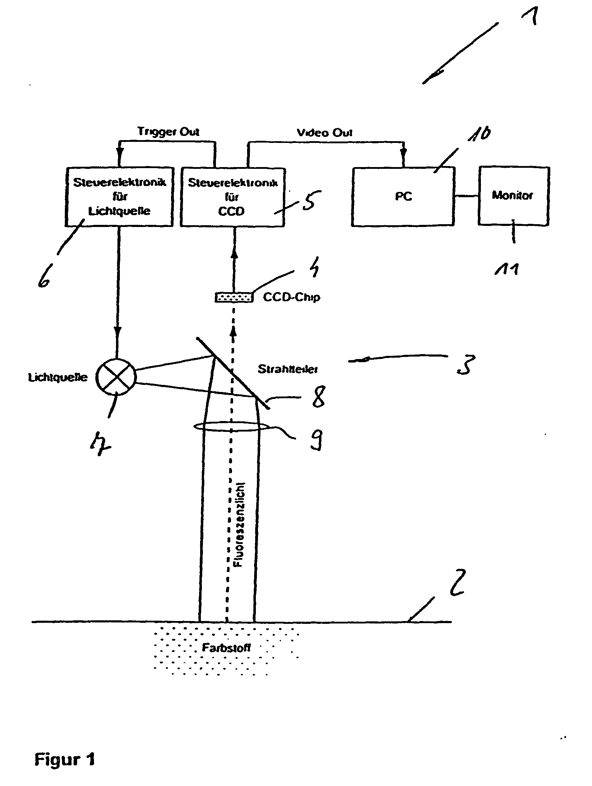

- Fig. 1: eine schematische Darstellung eines Systems für die Fluoreszenzdiagnostik von humanen Tumoren gemäß der Erfindung;
- Fig. 2: der spektrale Bereich der Absorption bzw. der Emission bei Verwendung von Protoporphyrin IX.

Das System 1 umfaßt u.a. ein digitales Kamerasystem 3 mit einem 3-CCD-Chip 4 (auch RGB-Chip) als optoelektrischen Bildwandler, eine erste Steuerelektronik 5 für das Kamerasystem 3 bzw. den 3-CCD-Chip 4, eine zweite Steuerelektronik 6 für eine gepulste Lichtquelle 7, die das Anregungslicht liefert und beispielsweise von wenigstens einer LED oder von einer Blitzlampe, z. B. Xenon-Hochdrucklampe, oder von einer Laserdiode oder Laserdiodenanordnung gebildet ist.

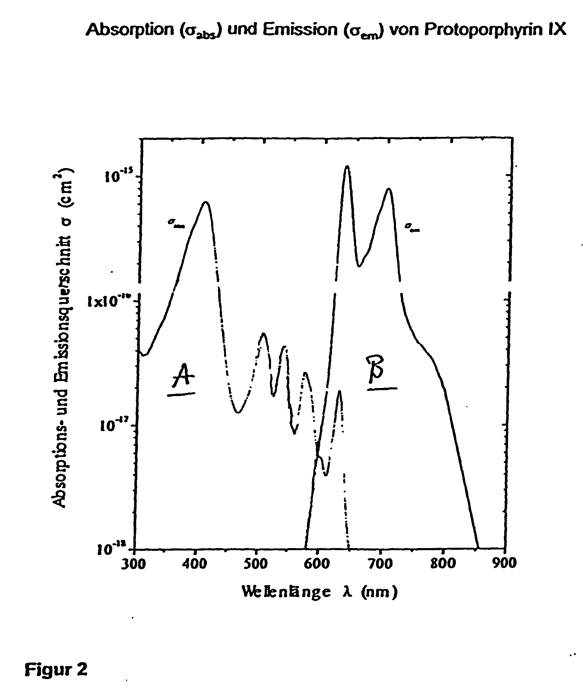

Wie die Figur 2 zeigt, liegt bei Verwendung von Porphyrinen als Fluoreszenzfarbstoff die maximale Absorption für die Anregung im blauen Spektralbereich (ca. 400 nm - Bereich A) und die maximale Fluoreszenzemission im roten Spektralbereich (ca. 630 nm - Bereich B).

Im Strahlengang bzw. in der optischen Achse des Kamerasystems 3 befindet sich ein Strahlteiler, der bei der dargestellten Ausführungsform von einem Teilerspiegel 8 gebildet ist und über den das von der Lichtquelle 7 gelieferte gepulste Anregungslicht in der optischen Achse des Kamerasystems 3 und durch die von einer Linse dargestellte Optik 9 dieses Systems auf das zu untersuchende Objekt oder Gewebeareal 2 aufgebracht wird. Der Strahlteiler 8 ist so ausgebildet und angeordnet, daß das Fluoreszenzbild von zu untersuchendem Gewebeareal 2 durch die Optik 9 und den Strahlteiler 8 hindurch auf den CCD-Chip auftrifft bzw. in der dortigen Bildebene abgebildet wird. Durch den Strahlteiler 8 wird auf diese Weise das Anregungslicht und das Fluoreszenzlicht getrennt.

Der Videoausgang der Steuerelektronik ist mit einem Rechner 10 verbunden, dem ein Monitor 11 zur Visualisierung der vom Kamerasystem 3 gelieferten Bilder, nämlich des normalen Farbbildes (auch RGB-Bild) sowie des Fluoreszenzbildes des zu untersuchenden Objektes 2 zugeordnet ist und der u.a. auch die für die Bilddarstellung auf den Monitor 11 notwendigen Komponenten, insbesondere auch Bildspeicher enthält. Dem Rechner 10, der beispielsweise von einem PC gebildet ist, sind noch weitere, nicht dargestellte Komponenten zugeordnet, nämlich die übliche Tastatur sowie Laufwerke für Speichermedien, wie Disketten, Bänder, Daten-CDs usw. Weiterhin sind dem Rechner 10 beispielsweise auch Mittel zugeordnet, über die eine Verbindung zu Datennetzwerken bzw. ein Datenaustausch über solche Netzwerke möglich ist.

Die Steuerelektronik 5 weist u.a. auch einen Trigger-Ausgang auf, mit dem die Steuerelektronik 6 zur synchronen Ansteuerung der Lichtquelle 7 getriggert wird. Mit dem beschriebenen System 1 ist es somit möglich, über das Kamerasystem 3 bei Umgebungsbeleuchtung, beispielsweise durch Tageslicht oder nicht dargestellte Lampen ein Normalbild von dem zu untersuchenden Gewebeareal 2 zu erhalten sowie durch von der Steuerelektronik 5 gesteuerte Triggerung der Steuerelektronik 6 und damit der Lichtquelle 7 für das Anregungslicht auch ein kurzzeitiges Fluoreszenzlichtbild. Zur Erzeugung des Fluoreszenzlichtbildes werden von der Steuerelektronik 5 zum Zeitpunkt der Aktivierung der Lichtquelle 7 und damit der Fluoreszenz (Aktivierungs- und Fluoreszenz-Phase) nur diejenigen Bildsignale des Kamerasystems 3 ausgewertet, die in dem dem Spektralbereich der Fluoreszenz entsprechenden Kanal des CCD-Chip anstehen, d.h. beispielsweise bei Verwendung von Protoporphyrin als Fluoreszenzfarbstoff das Signal des R-Kanals (Kanal für das rote Farbsignal).

Durch eine entsprechend kurze Aufnahmezeit für die Fluoreszenzbilder kann das Umgebungslicht aus diesen Bildern soweit diskriminiert werden, daß das System bei ständigem Umgebungslicht, d. h. auch bei während der Anregungs- und Fluoreszenzphase vorhandenem Umgebungslicht eindeutig arbeitet.

Weitere entscheidende Vorteile des Systems bestehen darin, daß das Fluoreszenzbild und das normale RGB-Bild des gesamten Beobachtungsareals 2 unmittelbar nacheinander detektiert, digitalisiert und in dem Rechner 10 verarbeitet werden, so daß die Möglichkeit besteht, beide Bilder unmittelbar nebeneinander oder aber auch übereinander am Monitor 11 darzustellen, so daß erkrankte Gewebeareale sich ohne Verlust an Information und Orientierung im RGB-Bild darstellen lassen.

Mittels einer im Rechner 10 durchgeführten Schwellwertberechnung lassen sich zusätzlich auch Grenzen zwischen gesundem und erkranktem Gewebe deutlich hervorheben. Wichtige Bilder können im Rechner 10 oder in Speichermedien abgespeichert und mit Daten des jeweiligen Patienten aktiviert werden, beispielsweise für eine Kontrolle therapeutischer Maßnahmen und/oder des Krankheitsverlaufs.

Die Erfindung wurde voranstehend an einem Ausführungsbeispiel beschrieben. Es versteht sich, daß zahlreiche Änderungen möglich sind, ohne daß dadurch der der Erfindung zugrundeliegende Erfindungsgedanke verlassen wird. So ist es beispielsweise auch möglich, die Lichtquelle 7 für das Anregungslicht so anzuordnen, daß dieses Licht nicht in den Strahlengang des Kamerasystems 3 eingeblendet wird, sondern außerhalb dieses Strahlungsganges auf das zu untersuchende Objekt 2 auftrifft. In diesem Fall ist dann im Strahlengang des Kamerasystems 3 anstelle des Strahlteilers 8 vorzugsweise ein Langpaßfilter (λ > 460 nm) vorgesehen, um das Fluoreszenzlicht vom Anregungslicht der Lichtquelle 7 zu trennen.

Vorstehend wurde weiterhin davon ausgegangen, daß mit einem einzigen Kamerasystem 3 bzw. mit einem einzigen CCD-Chip (RGB-Chip) sowohl das normale RGB-Bild, als auch das Fluoreszenzbild generiert werden.

### Bezugszeichenliste

- 1: System für die Fluoreszenzdiagnostik
- 2: Objekt bzw. Gewebe
- 3: Kamerasystem
- 4: 3-CCD-Chip
- 5, 6: Steuerelektronik
- 7: Lichtquelle für Anregungslicht
- 8: Strahlteiler
- 9: Kameraoptik
- 10: Rechner
- 11: Monitor

## Patentansprüche

1. System für die Fluoreszenzdiagnostik von Organen oder humanen Geweben mit wenigstens einer Anregungslichtquelle (7) zur Beaufschlagung eines Beobachtungsareals (2) mit einem Anregungslicht für eine Fluoreszenz, die aufgrund des Anregungslichtes durch einen im Gewebe vorhandenen Fluoreszenzfarbstoff erzeugt wird, und mit einem digitalen Kamerasystem (3),
bei dem die Anregungslichtquelle (7) eine gepulste Lichtquelle (7) ist, die zur Erzeugung eines gepulsten Anregungslichtes während einer Anregungs- und Fluoreszenzphase ausgebildet ist,
bei dem das digitale Kamerasystem (3) einen einzigen optoelektrischen Bildwandler (4) sowohl zur Generierung eines Fluoreszenzbildes als auch zur Generierung eines normalen RGB-Farbbildes bei Umgebungsbeleuchtung aufweist,
bei dem dem optoelektrischen Bildwandler (4) eine erste Steuerelektronik (5) zugeordnet ist, die von dem optoelektrischen Bildwandler (4) gelieferte Bildsignale als Bildsignale des normalen RGB-Farbbildes bzw. als Bildsignale des Fluoreszenzbildes synchron mit dem Aktivieren der wenigstens einen gepulsten Lichtquelle (7) aufnimmt und hieraus Bilder für eine weitere Bildbe- und/oder - verarbeitung generiert,
bei dem das normale RGB-Farbbild und das Fluoreszenzbild zeitlich nacheinander generiert werden, und zwar ein normales RGB-Farbbild außerhalb der Anregungs- und Fluoreszenzphase und zur Diskriminierung der während der Anregungs- und Fluoreszenzphase vorhandenen Umgebungsbeleuchtung ein kurzzeitiges Fluoreszenzbild während der Anregungs- und Fluoreszenzphase, und
bei dem der gepulsten Lichtquelle (7) eine zweite Steuerelektronik (6) zugeordnet ist, die von der ersten Steuerelektronik (5) zur synchronen Ansteuerung der Lichtquelle (7) derart getriggert ist.

2. System nach Anspruch 1, **dadurch gekennzeichnet, dass** der optoelektrische Bildwandler (4) einen RGB-Ausgang aufweist, und dass die erste Steuerelektronik (5) zur Generierung des Fluoreszenzbildes nur Bildsignale auswertet, die an dem dem Spektralbereich des Fluoreszenzbildes entsprechenden Ausgang des optoelektrischen Bildwandlers (4) anliegen.

3. System nach Anspruch 1 oder 2, **gekennzeichnet durch** Mittel (8) zum Einkoppeln des gepulsten Anregungslichtes der wenigstens einen gepulsten Lichtquelle (7) in den Strahlengang oder in die optische Achse des Kamerasystems (3).

4. System nach Anspruch 3, **dadurch gekennzeichnet, dass** die Mittel von wenigstens einem Teilerspiegel (8) gebildet sind.

5. System nach einem der vorhergehenden Ansprüche, **gekennzeichnet durch** wenigstens einen Filter im Strahlengang des Kamerasystems zur Ausfilterung des gepulsten Anregungslichtes.

6. System nach einem der vorhergehenden Ansprüche, **dadurch gekennzeichnet, dass** der optoelektrische Bildwandler (4) ein CCD-Chip ist.

7. System nach einem der vorhergehenden Ansprüche, **dadurch gekennzeichnet, dass** zur Generierung des Fluoreszenzbildes die erste Steuerelektronik das Signal des R-Kanals des optoelektrischen Bildwandlers (4) auswertet.

8. System nach einem der vorhergehenden Ansprüche, **dadurch gekennzeichnet, dass** das Spektrum des Lichtes der gepulsten Lichtquelle (7) im sichtbaren Spektralbereich und/oder im Infrarotbereich und/oder im UV-Bereich liegt.

## Claims

1. System for fluorescent diagnosis of organs or human tissues with at least one exciting light source (7) for scanning an observation area (2) with an exciting light for a fluorescence, which is produced by means of a fluorescent dye present in the tissue because of the exciting light, and with a digital camera system (3),
in which the exciting light source (7) is a pulsed light source (7), which is designed to produce a pulsed exciting light during an excitation and fluorescence phase,
in which the digital camera system (3) has a unique opto-electrical image converter (4) both for generating a fluorescent image and for generating a normal RGB colour image with ambient lighting, in which a first control logic unit (5), which receives image signals delivered by the opto-electrical image converter (4) as image signals in a normal RGB colour image or as image signals in the fluorescent image synchronously with the activation of at least one pulsed light source (7), is assigned to the opto-electrical image converter (4) and from these generates images for further image editing and/or processing, in which the normal RGB colour image and the fluorescent image are generated sequentially, namely a normal RGB colour image outside the exciting and fluorescence phase and for discrimination of the ambient light level present during the excitation and fluorescence phase a momentary fluorescent image during the excitation and fluorescence phase, and in which a second control logic unit (6), which is triggered in this way by the first control logic unit (5) for synchronous control of the light source (7), is assigned to the pulsed light source (7) .

2. System according to claim 1, **characterized in that** the opto-electrical image converter (4) has a RGB output and **in that** the first control logic unit (5) for generating the fluorescent image analyzes only image signals which are present at the output from the opto-electrical image converter (4) corresponding to the spectral range of the fluorescent image.

3. System according to claim 1 or 2, **characterized by** means (8) for coupling the pulsed exciting light from at least one pulsed light source (7) in the optical path or in the optical axis of the camera system (3).

4. System according to claim 3, **characterized in that** the means are formed by at least one beam splitter (8).

5. System according to one of the preceding claims, **characterized by** at least one filter in the optical path of the camera system for filtering out the pulsed exciting light.

6. System according to one of the preceding claims, **characterized in that** the opto-electrical converter (4) is a CCD chip.

7. System according to one of the preceding claims, **characterized in that** the first control logic unit (5) analyzes the signal from the R-channel of the opto-electrical converter (4) to generate the fluorescent image.

8. System according to one of the preceding claims, **characterized in that** the spectrum of the light from the pulsed light source (7) is in the visible spectrum and/or in the infrared spectrum and/or in the UV spectrum.

## Revendications

1. Système pour le diagnostic de la fluorescence d'organes ou de tissus humains, avec au moins une source de lumière d'excitation (7), pour soumettre une aire d'observation (2) à une lumière d'excitation pour une fluorescence produite par un colorant de fluorescence présent dans le tissu, au moyen de la lumière d'excitation, et avec un système d'appareil photo numérique (3),
dans lequel la source de lumière d'excitation (7) est une source de lumière pulsée (7), conçue pour la production d'une lumière d'excitation pulsée pendant une phase d'excitation et de fluorescence,
dans lequel le système d'appareil photo numérique (3) comporte un seul convertisseur d'image opto-électrique (4), aussi bien pour générer une image fluorescente que pour générer une image couleur RGB normale avec un éclairage ambiant,
dans lequel une première électronique de commande (5) est attribuée au convertisseur d'image opto-électrique (4), recevant des signaux d'image fournis par le convertisseur d'image opto-électrique (4), en tant que signaux d'image de l'image couleur RGB normale ou en tant que signaux d'image de l'image fluorescente, en synchronisation avec l'activation de l'au moins une source de lumière pulsée (7), et générant ainsi des images pour un nouveau traitement et/ou une nouvelle transformation d'image,
dans lequel l'image couleur RGB normale et l'image fluorescente sont générées l'une à la suite de l'autre dans le temps, en particulier une image couleur RGB normale en dehors de la phase d'excitation et de fluorescence, et une image fluorescente rapide pendant la phase d'excitation et de fluorescence, pour la distinction de l'éclairage ambiant présent pendant la phase d'excitation et de fluorescence, et
dans lequel une deuxième électronique de commande (6), déclenchée par la première électronique de commande (5) pour la commande synchrone de la source de lumière (7), est attribuée à la source de lumière pulsée (7).

2. Système selon la revendication 1, **caractérisé en ce que** le convertisseur d'image opto-électrique (4) comporte une sortie RGB, et **en ce que** pour la génération de l'image fluorescente, la première électronique de commande (5) n'évalue que les signaux d'image touchant la sortie du convertisseur d'image opto-électrique (4) correspondant à la région spectrale de l'image fluorescente.

3. Système selon l'une des revendications 1 ou 2, **caractérisé par** des moyens (8) pour l'intégration de la lumière d'excitation pulsée de l'au moins une source de lumière pulsée (7) dans le parcours optique ou dans l'axe optique du système d'appareil photo (3).

4. Système selon la revendication 3, **caractérisé en ce que** les moyens sont formés par au moins un miroir partiel (8).

5. Système selon l'une des revendications précédentes, **caractérisé par** au moins un filtre dans le parcours optique du système d'appareil photo, pour le filtrage de la lumière d'excitation pulsée.

6. Système selon l'une des revendications précédentes, **caractérisé en ce que** le convertisseur d'image opto-électrique (4) est une puce CCD.

7. Système selon l'une des revendications précédentes, **caractérisé en ce que** pour générer l'image fluorescente, la première électronique de commande évalue le premier signal du canal R du convertisseur d'image opto-électrique (4).

8. Système selon l'une des revendications précédentes, **caractérisé en ce que** le spectre de lumière de la source de lumière pulsée (7) se trouve dans la plage spectrale visible et/ou dans la plage infrarouge et/ou dans la plage UV.
